# EUROPEAN PATENT APPLICATION

(11) **EP 2 526 961 A1**
(43) Date of publication of application: **28.11.2012**
(21) Application number: 11167780.3
(22) Date of filing: 26.05.2011
(51) Int. Cl.: A61K 38/22, A61P 15/10, A61P 15/00

(54) **Treating or ameliorating some neuroendocrine disorders in male with help of prolactin**

(71) Applicant: Sargentini, Mario, 55100 Lucca (IT)
(72) Inventor: Sargentini, Mario, 55100 Lucca (IT)

(57) **Abstract**

Methods, compositions and kits for treating or ameliorating the following disorders, in male, with help of prolactin are disclosed: **a)** erectile dysfunction (ED), also from aging, of pituitary/hypothalamic origin; **b)** premature ejaculation (PE), of pituitary/hypothalamic origin. Said methods, compositions and kits comprise prolactin, variants, analogs, agonists and functional fragments of prolactin, also in combination with conventional therapies and drugs (eg testosterone/androgens and/or gonadotropins).

## Description

### Technical Field

The present invention concerns new therapeutic uses of prolactin and new methods, compositions, and kits comprising prolactin (PRL or LTH, luteotropic hormone) for the therapeutic treatment of the following disorders (when the prolactin level in blood is low or otherwise inadequate): **1) erectile dysfunction (ED), also from aging, in male, of pituitary/hypothalamic origin; 2) premature ejaculation (PE), in male, of pituitary/hypothalamic origin**, and for treating or ameliorating dysfunctions associated with such disorders.

Methods include the administration of prolactin, of variants, analogs, agonists and/or functional fragments of prolactin, also, possibly, in combination with conventional therapies and drugs. Conventional therapies and drugs are those normally used to treat the aforesaid disorders. Examples of conventional therapies are as follows: **a)** testosterone/ androgens, in various forms, and/or gonadotropins, to treat ED of pituitary/ hypo-thalamic origin; **b)** gonadotropins (FSH, LH, HCG, etc.) to treat infertility of pituitary/hypo-thalamic origin. The category "androgens" also includes anabolic steroids.

The methods and compositions relate to the use of prolactin, of variants, analogs, agonists and functional fragments of prolactin.

### Background Art

**Introduction**

Prolactin (PRL or LTH, luteotropic hormone) is a protein that in humans is encoded by the PRL gene. [EVANS, AM, et al. Mapping of prolactin and tumor necrosis factor-beta genes on human chromosome 6p using lymphoblastoid cell deletion mutants. Somatic Cell and Molecular Genetics . May 1989, vol.15, no.3, p.203-213.]

Prolactin is a polypeptide neuroendocrine hormone best known for his role in lactation. In breastfeeding, the act of an infant suckling the nipple stimulates the production of oxytocin which stimulates the "milk let-down" reflex [BARTHOLOMEW, Edwin F., et al. Essentials of anatomy & physiology. San Francisco: PEARSON/BENJAMIN CUMMINGS, 2007. ISBN 0805373039. p.340.], which fills the breast with milk via a process called lactogenesis, in preparation for the next feed.

It is secreted mainly by specialized cells (called "lactation cells") located at the front of the pituitary gland (adenohypophysis), of which constitute about 20%. In a lesser extent, however, PRL is also produced by: placenta, brain, uterus, dermal fibroblasts, cells of the deciduas, B lymphocytes, T lymphocytes, NK cells of the immune system and cells of breast cancer, and others.

Indeed it has been shown that in the immune system of the human race, prolactin is secreted by mono-nucleated cells of peripheral blood (PBMC) (as lymphocytes, monocytes and macrophages) and that it acts as an agent of proliferative growth. It has also been shown that treatment of PBMC with prolactin increases the production of IFNγ (interferon γ). PRL has metabolic and anabolic effects on protein synthesis, anti insulin and adipokinetic effects similar to those of pituitary growth hormone (GH). Indeed PRL is substantially a factor of cell growth, similar to GH. The intimate mechanisms by which the PRL exerts the action of on target cells are subject to further clarification.

Pituitary prolactin secretion is regulated by neuroendocrine neurons in the hypothalamus, the most important ones being the neurosecretory tuberoinfundibulum (TIDA) neurons of the arcuate nucleus, which secrete dopamine to act on the dopamine-2 receptors of lactotrophs, causing inhibition of prolactin secretion. Thyrotropin-releasing factor (thyrotropin-releasing hormone) has a stimulatory effect on prolactin release. The pituitary hormone prolactin is the only one who suffers a constant inhibitory tone by the hypothalamus.

Vasoactive intestinal peptide (VIP) and peptide histidine isoleucine help to regulate prolactin secretion in humans, but the functions of these hormones can be quite different in other species (as, for example, in birds). [KULICK, R, et al. The relative importance of vasoactive intestinal peptide and peptide histidine isoleucine as physiological regulators of prolactin in the domestic turkey. Generaland Comparative Endocrinology. July 2005, vol.142, no.3, p.267-273.]

Prolactin is sometimes classified as a gonadotropin [**MARIEB, E.N., et al.** Human Physiology & Anatomy. 7th edition. London: Benjamin Cummings, 2006. p.605.] although the dominant view is that in humans it has only a weak luteotropic effect while the effect of suppressing classical gonadotropic hormones is more important. **[National Library of Medicine-Medical Subject Headings - 2009 MeSH - MeSH Descriptor Data -** http ://www.nlm.nih.gov/cgi/mesh/2009/MB cgi?mode=&term=Gonadotropins]

**Effects**

Prolactin has many effects including regulating lactation and stimulating proliferation of oligodendrocyte precursor cells. It stimulates the mammary glands to produce milk (lactation): Increased serum concentrations of prolactin during pregnancy cause enlargement of the mammary glands of the breasts and prepare the production of milk. However, the high levels of progesterone during pregnancy supress the production of milk. Milk production normally starts when when the levels of progesterone fall by the end of pregnancy and a suckling stimulus is present. Sometimes, newborn babies (males as well as females) secrete a milky substance from their nipples known as witch's milk. This is in part caused by maternal prolactin and other hormones. Prolactin provides the body with sexual gratification after sexual acts: The hormone counteracts the effect of dopamine, which is responsible for sexual arousal. This is thought to cause the sexual refractory period. The amount of prolactin can be an indicator for the amount of sexual satisfaction and relaxation. Unusually high amounts (hyperprolactinemia) are suspected to be responsible for impotence and loss of libido.

Prolactin also stimulates proliferation of oligodendrocyte precursor cells. These cells differentiate into oligodendrocytes, the cells responsible for the formation of myelin coatings on axons in the central nervous system. [ GREGG, C., et al. White matter plasticity and enhanced remyelination in the maternal CNS. J. Neurosci. 27(8): 1812-23*.* Feb. 2007, vol.21, no.27(8), p.1812-1823.]

Prolactin also has a number of other effects including contributing to surfactant synthesis of the fetal lungs at the end of the pregnancy and immune tolerance of the fetus by the maternal organism during pregnancy; hyper-prolactinemia also decreases normal levels of sex hormones-estrogens in women and testosterone in men. It is this inhibition of sex steroids that is responsible for loss of the menstrual cycle in lactating women as well as lactation-associated osteoporosis. Prolactin also enhances luteinizing hormone-receptors in Leydig cells, resulting in testosterone secretion which leads to spermatogenesis. Prolactin delays hair regrowth in mice. [CRAVEN, AJ, et al. Prolactin delays hair regrowth in mice. Journal of Endocrinology. Aug. 2006, vol.191, p.415-425.] Prolactin is also present in males but its role is not well understood.

**Variance in levels.**

There is a diurnal as well as an ovulatory cycle in prolactin secretion. In many mammals, there is also a seasonal change in prolactin release. During pregnancy, high circulating concentrations of estrogen and progesterone inhibit the action of prolactin on milk production. Following delivery, reduced estrogen and progesterone production allows prolactin to induce lactation. After childbirth, prolactin levels fall as the internal stimulus for them is removed. Sucking by the baby on the nipple then promotes further prolactin release, maintaining the ability to lactate. The sucking activates mechanoreceptors in and around the nipple. These signals are carried by nerve fibers through the spinal cord to the hypothalamus, where changes in the electrical activity of neurons that regulate the pituitary gland cause increased prolactin secretion. The suckling stimulus also triggers the release of oxytocin from the posterior pituitary gland, which triggers milk let-down: Prolactin controls milk production (lactogenesis) but not the milk-ejection reflex; the rise in prolactin fills the breast with milk in preparation for the next feed. In usual circumstances, in the absence of galactorrhea, lactation will cease within one or two weeks of the end of demand breastfeeding. It has also been found that compared to un-mated males, fathers and expectant fathers have increased prolactin concentrations. [NELSON, R.J. An introduction to behavioral endocrinology. 3rd edition. Sunderland: Sinauer Associates, 2005.]

High prolactin levels also tend to suppress the ovulatory cycle by inhibiting the secretion of both follicle-stimulating hormone (FSH) and gonadotropic-releasing hormone (GnRH). High prolactin levels can also contribute to mental health issues. Prolactin levels peak during REM sleep, and in the early morning. Levels can rise after exercise, meals, sexual intercourse, minor surgical procedures [MELMED, S. "333 Disorders of the Anterior Pituitary and Hypothalamus", in Jameson JN, Kasper DL, Harrison TR, Braunwald E, Fauci AS, Hauser SL, Longo DL.. Harrison's principles of internal medicine. 16th edition. New York: McGraw-Hill Medical Publishing Division, 2005. ISBN 0071402357.], or following epileptic seizures. [MELLORS, JDC, et al. The approach to patients with "non-epileptic seizures. Postgraduate Medical Journal. 2005, vol.81, no.958, p.498-504.] Hypersecretion of prolactin is more common than hyposecretion. Hyperprolactinemia is the most frequent abnormality of the anterior pituitary tumors. Clinical signs include inappropriate lactation, lack of menses, infertility in females and impotence in males (that is erectile dysfunction (ED)).

**Structure.**

Prolactin has different molecular forms. In humans and other mammals have been identified variants of the PRL, the majority of which are high molecular weight isoforms (macro-prolactins), due to processes such dimerization, polymerization and aggregation to other proteins, with varying degrees of glycosylation. In humans the main form of circulating PRL is a a single chain monomer of 199 aminoacids: non-glycosylated form is 23 kDa and the glycosylated form is 25 kDa. Glycosylated prolactin is removed from circulation more rapidly and was reported to have less biological potency. Some sources report, however, a total of 198 amino acids components, with a molecular weight of about 24 kDa. The complementary DNA for prolactin (cDNA) encoding a protein of 227 amino acids with an N-terminal signal peptide of 28 amino acids. The human prolactin adopts a three-dimensional structure composed of four antiparallel helices.

Prolactin is commonly assayed in laboratories of hospital departments of endocrinology, in case of problems and endocrinological diseases involving the pituitary gland.

Its structure is similar to that of growth hormone and placental lactogen. The molecule is folded due to the activity of three disulfide bonds. Significant heterogeneity of the molecule has been described, thus bioassays and immunoassays can give different results due to differing glycosylation, phosphorylation, sulfation, as well as degradation. The non-glycosylated form of prolactin is the dominant form of prolactin that is secreted by the pituitary gland. Little prolactin is apparently the result of removal of some amino acids, whereas big prolactin can be the product of interaction of several prolactin molecules. Pit-1 is a transcription factor that binds to the prolactin gene at several sites to allow for the production of prolactin in the pituitary gland. A key regulator of prolactin production is estrogens that enhance growth of prolactin-producing cells and stimulate prolactin production directly, as well as suppressing dopamine. Human prolactin receptors are insensitive to mouse prolactin. [UTAMA, FE, et al. Human prolactin receptors are insensitive to mouse prolactin: implications for xenotransplant modeling of human breast cancer in mice. Journal of Endocrinology. 2006, vol.188, p.589-601.]

**Prolactin receptor.**

Prolactin receptors are present in: mamillary glands, cells of the sexual organs (both male and female), cells of immune system, pituitary gland, heart, lung, thymus, spleen, liver, pancreas, kidney, adrenal gland, skeletal muscle, skin and areas of the central nervous system. [MANCINI, T, et al. Hyperprolactinemia and Prolactinomas. Endocrinology and Metabolism Clinics of North America. March 2008, vol.37, no.1, p.67-99.] When prolactin binds to the receptor, it causes it to dimerize with another prolactin receptor. This results in the activation of Janus kinase 2, a tyrosine kinase which initiates the JAK-STAT pathway. The activation of the prolactin receptor also results in the activation of mitogen-activated protein kinases and Src kinase.

**Diagnostic use.**

Prolactin levels may be checked as part of a sex hormone workup, as elevated prolactin secretion can suppress the secretion of FSH and GnRH, leading to hypogonadism, and sometimes causing erectile dysfunction in men. Prolactin levels may be of some use in distinguishing epileptic seizures from psychogenic non-epileptic seizures. The serum prolactin level usually rises following an epileptic seizure. **[**BANERJEE, S, et al. Serum prolactin in seizure disorders. Indian Pediatr.. Aug. 2004, vol.41, no.8, p.827-31.]

**Reference Levels.**

**Table 1**

| | ng/mL | mIU/L | Prolactin Standard |
|---|---|---|---|
| Women | 2.8-29.2 | 59-619 | (World Health Organization [WHO]: Third International Reference Preparation [3rd IRP] of prolactin, human, lyophilized, 0.053 IU / ampoule, for immunoassay [preparation code:84/500] [ **WHO Expert Committee on Biological Standardization.** Thirty-ninth Report, WHO Technical Report Series. Geneva: World Health Organization - Technical Report 786, 2009.] |
| Men | 2.1-17.7 | 45-375 | (WHO: 3rd IRP 84/500) |

(Data from The Immunoassay Handbook, Third Edition) [WILD, D. The Immunoassay Handbook. 3rd edition. Kidlington, Oxford: Elsevier Science, 2005. ISBN 0080445268. p.930.]

When measured under normal conditions in the morning most values fall into the range 4-12 ng/mL for women and 2-4 ng/mL for men. Measurement at other times of the day or after stress exposure will yield different values.

To date the human PRL hormone is available as a reagent, also in DNA recombinant form, for use in laboratory research on cell cultures (and, de facto, even on animals). To date prolactin is not available, in the world, for human use and it has no therapeutic indication for the disorders mentioned in this patent application.

**Background Art. Further details.**

**Pathophysiology.**

Prolactin deficiency is characterized by the inability of pituitary lactotrophs to secrete prolactin.

In the vast majority of prolactin deficiency states, the deficiency occurs secondary to general anterior pituitary dysfunction. The most commonly associated condition is postpartum pituitary necrosis (Sheehan syndrome); however, prolactin deficiency can also be caused by anterior pituitary impairment secondary to pituitary (or extrapituitary) tumor or treatment of tumor, parasellar diseases, head injury, empty sella syndrome; infection (eg, tuberculosis, histoplasmosis), or infiltrative diseases (eg, sarcoidosis, hemochromatosis, lymphocytic hypophysitis) **[**COSMAN, F, et al. Lymphocytic hypophysitis. Report of 3 new cases and review of the literature. Medicine (Baltimore). Jul 1989, vol.68, no.4, p.240-56.][ THODOU, E, et al. Clinical case seminar: lymphocytic hypophysitis: clinicopathological findings. J Clin Endocrinol Metab. Aug. 1995, vol.80, no.8, p.2302-11.] **[**TOLEDANO, Y, et al. Acquired prolactin deficiency in patients with disorders of the hypothalamic-pituitary axis. J Endocrinol Invest. Apr. 2007, vol.30, no.4, p.268-73.]

Other pathophysiologic mechanisms have not been fully established and understood, so as the endocrine and metabolic function of prolactin.

The dominant view is that the clinical manifestation of prolactin deficiency only occur in females and is probably limited to puerperal alactogenesis. [ ZARGAR, AH, et al. Familial puerperal alactogenesis: possibility of a genetically transmitted isolated prolactin deficiency. Br J Obstet Gynaecol. May 1997, vol.104, no.5, p.629-31.]

However menstrual disorders, delayed puberty, infertility, and subfertility have been associated with hypoprolactinemia, through mechanisms that are not entirely clear. Prolactin concentration in follicular fluid during in vitro fertilization (IVF) correlates with the oocyte maturation level and fertilization rate. Further, in a randomized human trial, bromocriptine-induced hypoprolactinemia during IVF resulted in decreased fertilization and cleavage rate compared with a hyperprolactinemic cycle group. A partial prolactin deficiency may result in inadequate lactation. Further, a possibility exists that male factor infertility may be associated with hypoprolactinemia.

Prolactin, as well as the GH, does not target specific organs, but has its effects on a large number of cells and systems, including the sexual organs, in male and female. The action of prolactin seems to be mainly a trophic action on the receptors for other hormones and substances. So if these receptors are not working, the action of these other hormones and substances is not effective.

An isolated prolactin deficiency is rare. More frequently we have deficit of prolactin in hypopituitarism and panhypopituitarism. Indeed, the prolactin assay is used to test the degree of hypopituitarism. Most often still there are cases of partial deficiency, where the dosage of the hormone remains in the lower part of the normal range. Causes of deficiency of the hormone involve the pituitary gland primitively (primitive hypopituitarism) and are the following [http://www.msd-italia.it/altre/manuale/tabelle/00701 .html] :

*** Causes affecting the pituitary gland primitively (primitive hypopituitarism):**

- Pituitary tumors: Adenomas; Craniopharyngiomas;

- Infarction or ischemic necrosis of the pituitary: Shock, particularly post-partum (Sheehan's syndrome) or diabetes mellitus or sickle cell anemia; Vascular thrombosis or aneurysms, especially the internal carotid artery; Hemorrhagic infarction (pituitary apoplexy);

- Inflammatory: Meningitis (tuberculous, bacterial, fungal, malarial); Pituitary abscess; Sarcoidosis;

- Infiltrative disease: Langerhans cell histiocytosis (Hand-Scholler disease-Christian); Hemochroma-tosis;

- Idiopathic pituitary hormone deficiency, isolated or multiple;

- Empty sella syndrome;

- latrogenic causes: Irradiation; Surgical Ablation;

- Autoimmune disorders of the pituitary (lymphocytic hypophysitis);

* **Causes affecting the hypothalamus primitively (secondary hypopituitarism):**

- Hypothalamic tumors: Pinealomas; Meningiomas; Ependymomas; Metastatic tumors;

- Inflammatory processes, such as sarcoidosis;

- Trauma (sometimes associated with fracture of the skull base);

- Neurohormonal hypothalamic deficiency, isolated or multiple;

- Surgical section of the pituitary stalk.

Some data support the idea that prolactin is also an immunoregulating hormone. Prolactin receptors have been found on human T lymphocytes and B lymphocytes, and some data support T-lymphocyte dependence on prolactin for maintenance of immune competence. [RUSSELL, DH, et al. Prolactin receptors on human T and B lymphocytes: antagonism of prolactin binding by cyclosporine. J Immunol.. May 1985, vol.134, no.5, p.3027-31.]

In research using a mouse model, inhibition of prolactin release impaired lymphocyte function and depressed macrophage activation. **[**BERNTON, EW, et al. Suppression of macrophage activation and T-lymphocyte function in hypoprolactinemic mice. Science. Jan 1988, vol.239, no.4838, p.401-4.] Further, the study's mice had a decreased tolerance for bacterial exposure; this reduced tolerance was manifested by death from a normally nonlethal dose of bacteria.

Part of the immunosuppressive effects of cyclosporine may be mediated through a competitive antagonistic action at the prolactin receptor site. Further evidence is found in the observation of the immunosuppressant effects of bromocriptine, which has been shown to be an effective adjuvant (immunosuppressant) in patients after transplantation and in patients with autoimmune disease. [CARRIER, M, et al. Bromocriptine as an adjuvant to cyclosporine immunosuppression after heart transplantation. Ann Thorac Surg.. Jan. 1990, vol.49, no.1, p.129-32.][ PALESTINE, AG, et al. Therapy for human autoimmune uveitis with low-dose cyclosporine plus bromocriptine. Transplant Proc.*.* Jun 1988, vol.20, no.3 Supp. 4, p.131-5.] . The effects of prolactin on immune system are object of some patents.

Because prolactin release is inversely related to dopamine levels in the anterior pituitary, critically ill patients on prolonged dopamine infusion have resultant prolactin deficiency. It has been hypothesized that this causes impairment of the T-lymphocyte proliferation response; this impairment occurs in patients in intensive care units (ICUs) and may be an important cause of infection susceptibility in this group. However, no data support the hypothesis that lack of prolactin in otherwise healthy patients results in immunodeficiency.

Several studies have found a correlation in preterm infants between hypoprolactinemia and increased mortality. [LUCAS, A, et al. Plasma prolactin and clinical outcome in preterm infants.. Arch Dis Child. Sep 1990, vol.65, no.9, p.977-83.] The precise pathophysiologic mechanism is unknown, but it is speculated to be associated with the effects of prolactin on surfactant synthesis, whole-body water regulation, or gastrointestinal maturation. [BONOMO, IT, et al. Prolactin inhibition in dams during lactation programs for overweight and leptin resistance in adult offspring. J. Endocrinol. Feb. 2007, vol.192, no.2, p.339-44.]

### Summary of invention

The dominant view is that the neuroendocrine clinical manifestation of prolactin deficiency only occur in females and is probably limited to puerperal alactogenesis. Described herein are instead methods, compositions, and kits for treating, ameliorating, or for prophylactically addressing symptoms of the following neuroendocrine disorders in males:
**1) erectile dysfunction (ED),** also from aging, of pituitary/hypothalamic origin, and **2) premature ejaculation (PE),** of pituitary/hypothalamic origin, and for treating or ameliorating dysfunctions associated with such disorders.

Methods include the administration of prolactin, of variants, analogs, agonists and/or functional fragments of prolactin, also, possibly, in combination with conventional therapies and drugs. Conventional therapies and drugs are those normally used to treat the aforesaid disorders. Examples of conventional therapies are as follows: **a)** testosterone/ androgens, in various forms, and/or gonadotropins, to treat ED; **b)** gonadotropins (FSH, LH, HCG, etc.) to treat infertility of pituitary/hypo-thalamic origin. The category "androgens" also includes anabolic steroids.

In the case of an isolated prolactin deficiency is sufficient to administrate prolactin. In the case of hypopituitarism or pan-hypopituitarism, disorders normally associated also with low levels of prolactin and gonadotropins, the combined administration of **prolactin** and **testosterone/androgens,** and/or **gonadotropins,** has one or more combinatorial or synergistic effects in reducing dysfunctions associated with such disorders.

Compositions and kits including prolactin and testosterone/androgens, and/or gonadotropins, are also provided. The composition is optionally a pharmaceutical composition for use in treating or ameliorating the aforesaid neuroendocrine disorders. Thus provided herein is a use for a composition including prolactin (or variants, analogs, agonists and/or functional fragments of prolactin) and testosterone/androgens, and/or gonadotropins, for the manufacture of medicaments for treating or ameliorating the aforesaid neuroendocrine disorders.

### Description of embodiments

The invention concerns new and surprising therapeutic uses of prolactin (PRL or LTH, luteotropic hormone) in males, and methods, compositions, and kits for treating, ameliorating, or for prophylactically addressing symptoms of some neuroendocrine disorders in males. Disorders caused by hyperprolactinaemia are well known, but much less known, studied and understood, even for the lower statistical frequency, are disorders caused by hypoprolactinaemia. The dominant view is that the clinical manifestation of prolactin deficiency only occur in females and is probably limited to puerperal alactogenesis. However, some studies seem to indicate that prolactin deficiency can cause a lot of dysfunctions on sexual health, even in male. Prolactin, as well as the GH, does not target specific organs, but has its effects on a large number of cells and systems, including the sexual organs, in male and female. Disorders caused by hypoprolactinaemia result, essentially, from the fact that prolactin seems to have a permissive effect on the functioning of other important hormones (such as FSH and LH) and systems. The action of prolactin seems to be mainly a trophic action on the receptors for other hormones and substances. So if these receptors are not working, the action of these other hormones and substances is not effective.

Provided herein are methods, compositions and kits for treating or ameliorating, when the serum prolactin level is low or otherwise inadequate, the following disorders in male: **1) erectile dysfunction (ED), also from aging, of pituitary/hypothalamic origin; 2) premature ejaculation (PE), of pituitary/hypothalamic origin,** and for treating or ameliorating dysfunctions associated with such disorders.

The methods and compositions relate to the use of **prolactin**, of variants, analogs, agonists and functional fragments of prolactin, also, possibly, in combination with conventional therapies and drugs. Conventional therapies and drugs are those normally used to treat the aforesaid disorders. Examples of conventional therapies are as follows: **a)** testosterone/ androgens, in various forms, and/or gonadotropins, to treat ED; **b)** gonadotropins (FSH, LH, HCG, etc.) to treat infertility of pituitary/ hypothalamic origin.

In the case of an isolated prolactin deficiency is sufficient to administrate prolactin. In the case of hypopituitarism or pan-hypopituitarism, disorders normally associated also with serum low levels of prolactin and gonadotropins, the combined administration of **prolactin** and **testosterone/ androgens,** and/or **gonadotropins,** has one or more combinatorial or synergistic effects in reducing dysfunctions associated with such disorders.

We have also found that any replacement therapy to treat ED, for example testosterone/androgens or gonadotropins, does not work if the level of prolactin in blood is low or otherwise inadequate. We have found that, for sexual health, the serum level of prolactin must be adequate to the needs of organism, also in male.

Anyway, to date, prolactin (PRL) has, in the world, no therapeutic indications for the disorders mentioned in this patent application.

The invention includes new therapeutic uses of prolactin, also, possibly, in combination with conventional therapies and drugs (testosterone/ androgens and/or gonadotropins), for the therapeutic treatment of the aforesaid disorders (when the prolactin level in blood is low or otherwise inadequate), and for treating or ameliorating dysfunctions associated with such disorders. The methods and compositions relate to the use of prolactin, of variants, analogs, agonists and functional fragments of prolactin.

The invention includes methods and pharmaceutical compositions comprising prolactin, variants, analogs, agonists and functional fragments of prolactin, also, possibly, in combination with conventional therapies and drugs (testosterone/androgens and/or gonadotropins), for the therapeutic treatment of the aforesaid disorders, and for treating or ameliorating dysfunctions associated with such disorders, when the prolactin level in blood is low or otherwise inadequate.

The invention includes kits containing a composition comprising prolactin, or variant, analog, agonist or functional fragment of prolactin, and, possibly, conventional drugs (testosterone/androgens and/or gonadotropins), in the appropriate doses and combinations, in one or more containers. This composition is for the therapeutic treatment of the aforesaid disorders and for treating or ameliorating dysfunctions associated with such disorders, when the prolactin level in blood is low or otherwise inadequate. Any kit can include instructions for administering the composition to the subject and/or include a device for the same administration.

Variants, analogs, agonists and functional fragments of prolactin are substances, also possibly derived from prolactin, which have biological effects comparable or superior to those of prolactin. These substances can also be used in place of prolactin in pharmaceutical compositions and methods described in this patent application. The category "variants" includes also modified prolactin by the addition of molecules or end-groups that have particular effects, as, for example, extending half-life of the same prolactin in the blood, thus allowing less frequent administrations.

The aforementioned compositions may also comprise a pharmaceutically acceptable carrier and/or other agents (eg agents that increase the permeability of the blood-brain barrier). By pharmaceutically acceptable is meant a material that does not produce unwanted biological effects. Thus, the composition may be administered to a subject, without causing unacceptable biological effects or unacceptable interactions with any of the other components of the pharmaceutical composition. The carrier should minimize the deterioration of prolactin, and minimize any adverse reactions. Suitable pharmaceutical carriers are described in Remington: The Science and Practice of Pharmacy (21st ed.) ed. University of the Sciences in Philadelphia, Williams & Wilkins Lipincott 2005. Typically, it is used a pharmaceutically acceptable salt in suitable quantity to make the pharmaceutical composition isotonic. The composition may also include one or more active ingredients such as, for example, antimicrobial agents.

By subject is meant any human individual.

The agents and compositions above (eg, prolactin) may be administered: oral, parenteral (eg intravenous), intramuscular, intraperitoneal, transdermal, topically, by inhalation, subcutaneously, with administration in the central nervous system, or a combination of these pathways.

The dosage of the composition or agents may vary from subject to subject, depending on the species, age, weight and general condition of the subject, and on the way of administration. As a general rule, the doses to be administered are those sufficient to produce the desired effect on the symptoms of the disorder. The dosage should not however be so great as to cause adverse reactions. It should also be noted that lower doses of agents can be used in case of synergistic effects. The agents and compositions can be administered one or more days, weeks, months or years. Therefore, the duration of treatment may optionally be one or more days, weeks, months or years and may be continued throughout the life of the subject.

Treating or ameliorating means to reduce or completely remove one or more symptoms or signs of a disease, or delay the onset or recrudescence of symptoms or signs of a disease.

An effective dose of a therapeutic agent is a dose sufficient to achieve the intended purpose. The effective dose of a particular therapeutic agent depends on such factors as the nature of the agent, the route of administration, the weight and the purpose of administration. The effective dose for an individual patient can be determined empirically by a skilled physician according to proper medical practice.

When relative terms, how to improve, increase, decrease, reduce, and the like are used herein are generally used in reference to a level of control. For example, a level of control may be that of different subjects that do not have the disease, or a level of control may be the same subject, before and after initiation of treatment.

The agents and pharmaceutical compositions disclosed herein can be used in various combinations. Therefore, it is understood that the patent application covers the use of prolactin, variants, analogs, agonists and functional fragments thereof, in the aforementioned combinations. Thus, for example, in the case of an association between prolactin and a permeabilizer of the blood-brain barrier.

Throughout this application, various publications are referenced. The disclosures of these publications in their entireties are hereby incorporated by reference into this application in order to more fully describe the state of the art to which this invention pertains.

The findings are more particularly described in the following example which is intended as illustrative only, as numerous modifications and variations therein will be apparent to those skilled in the art.

### Example

To study the role played by normal levels of plasma prolactin (PRL) in the sexual health in male, the author of this patent application has tested the findings and the effects of hypoprolactinemia on himself. I decided to take a calculated risk, to realize the situation personally, because the evaluation of sexual health can be difficult, as it may be influenced by personal feelings and situations, including psychological.

I utilized Dostinex tablets 0,5 mg (active ingredient: cagergoline). I induced hypoprolactinemia on myself using Dostinex for 4 weeks. The first week I assumed one tablet of 0,5 mg every 3 days. The second and third week I assumed one tablet of 0,5 mg, every 2 days. The fourth week I assumed one tablet of 0,5 mg, every 3 days.

Early, during the trial, the following dysfunctions were manifested: erectile dysfunction and premature ejaculation. The seriousness of which gets progressively worse with continuation of therapy and with increasing the dose. The seriousness of dysfunctions became so pronounced that it was practically impossible to achieve and maintain erection, while, at the same time, ejaculation had become so premature that, in case of strong excitation, the same ejaculation was possible even before reaching the erection and without penetration.

To test the effect of hypoprolactinaemia on a testosterone replacement therapy I used 40 mg oral capsule Andriol (testosterone undecanoate), and then Testoviron 250 mg/ml solution for intramuscular injection, prolonged release (testosterone enanthate 250 mg).

Concomitant administration of Andriol capsules 40 mg (3 tablets a day for 5 days) did not improve at all the above dysfunctions.

The subsequent administration of Testoviron 250, one vial a week for 2 weeks, had minimal effects on the above-mentioned dysfunctions. At the laboratory evaluation Testoviron caused a slight increase of serum prolactin, but a simultaneous significant decrease of serum gonadotropins LH and FSH (0,5 mUI/mL LH (r.v.: 1,0 - 9,0) and 0,7 mUI/mL FSH (r.v.: 1,3 - 19,5)).

That confirm that the administration of testosterone, in various forms, is ineffective to treat ED and PE in presence of hypoprolactinaemia.

In healthy subjects the increase of prolactin induced by administration of anabolic steroids (such as Decadurabolin, active ingredient: nandrolone) is a known effect.

It is considered that in this case the effect of Testoviron on prolactin and on the aforesaid dysfunctions have been insufficient because of the simultaneous administration of Dostinex and because of the strong decrease of LH and FSH.

The only way to eliminate these dysfunctions was the discontinuation of Dostinex and restoring the normal level of prolactin in blood. To do this I help me using successfully Eutimil 20 mg (active ingredient: paroxetine): one tablet per day for about 2 weeks. After about 2 weeks of therapy with Eutimil I realized that no longer I needed it and I interrupted the treatment, without unexpected side effects. Paroxetine is an SSRIantidepressant, which can cause, as a side effect, a weak hyperprolactinemia.

As additional symptoms during therapy with Dostinex I had only a slight nausea and weakness, which are however consistent with the supervening ineffectiveness of androgens.

The findings confirm that normal levels of plasma PRL play an important role in the sexual health and in the effectiveness of androgens in vivo. The findings confirm also that normal levels of plasma PRL are necessary for the effectiveness of any replacement therapy with testosterone/ androgens and gonadotropins, to treat or ameliorate erectile dysfunction and premature ejaculation of pituitary/hypothalamic origin. From this the helpful of prolactin for the therapeutic treatment of the aforesaid disorders (when the prolactin level in blood is low or otherwise inadequate): erectile dysfunction and premature ejaculation, of pituitary/hypothalamic origin. Further trials are planned, however.

### Industrial applicability

A number of methods and compositions have been described. Nevertheless, it will be understood that various modifications may be made without departing from the spirit and scope of the invention. For example, the use of variants of prolactin instead of prolactin can be used in compositions in which only prolactin is recited. Accordingly, other embodiments are within the scope of the following claims.

## Claims

1. A method of treating or ameliorating a neuroendocrine disease/disorder in male human subject comprising: **a.** administering to the subject prolactin (or a variant, analog, agonist or functional fragment of prolactin) and **b.** administering to the subject testosterone/androgens, in various forms, and/or gonadotropins (LH, FSH, HCG, etc.). The category "androgens" also includes anabolic steroids.

2. The method of claim 1, wherein the disease/disorder is **erectile dysfunction (ED),** of pituitary/hypothalamic origin.

3. A method of treating or ameliorating a neuroendocrine disease/disorder in male human subject comprising: **a.** administering to the subject prolactin (or a variant, analog, agonist or functional fragment of prolactin) and **b**. administering to the subject testosterone/androgens, in various forms, and/or gonadotropins (LH, FSH, HCG, etc.).

4. The method of claim 3, wherein the disease/disorder is **premature ejaculation (PE)**, of pituitary/hypothalamic origin.

5. A composition comprising prolactin, or variant, analog, agonist, or functional fragment of prolactin. It is understood that any reference to prolactin is also referred to variants, analogs, agonists and active fragments of prolactin.

6. A kit for treating or ameliorating the aforesaid neuroendocrine disorders in a subject, comprising prolactin, or prolactin and testosterone/androgens, or prolactin and gonadotropins, in one or more containers.

7. The kit of claim 6, wherein the kit comprises at least one container with prolactin or a combination of prolactin and testosterone/androgens, or prolactin and gonadotropins.

8. The kit of claim 6, wherein the kit further comprises: **a**. instructions for administering to the subject prolactin or prolactin and testosterone/androgens, or prolactin and gonadotropins; b. at least one device for administering to the subject prolactin or prolactin and testosterone/androgens, or prolactin and gonadotropins.

9. A pharmaceutical composition comprising prolactin, or prolactin and testosterone/androgens, or prolactin and gonadotropins, for use in treating or ameliorating the aforesaid neuroendocrine disorders.

10. The pharmaceutical composition of claim 9, wherein the neuroendocrine disorders are the aforesaid: - erectile dysfunction (ED), in male, of pituitary/hypothalamic origin; - premature ejaculation (PE), in male, of pituitary/hypothalamic origin.

11. Use of a composition comprising prolactin, or prolactin and testosterone/ androgens, or prolactin and gonadotropins, for the manufacture of a medicament for treating or ameliorating the aforesaid neuroendocrine disorders.

12. The methods of claims 1 and 3, wherein prolactin, or prolactin and testoste-rone/androgens, or prolactin and gonadotropins, is administered to the subject.

13. The use and administration to the subject of **prolactin**, of variants, analogs, agonists and functional fragments of prolactin, for the therapeutic treatment, possibly also in combination with conventional therapies and drugs, of the following disorders (when the prolactin level in blood is low or otherwise inadequate): **a**. erectile dysfunction (ED), also from aging, in male, of pituitary/hypothalamic origin; **b**. premature ejaculation (PE), in male, of pituitary/hypothalamic origin, and for treating or ameliorating dysfunctions associated with such disorders.

14. Methods and pharmaceutical compositions comprising **prolactin**, variants, analogs, agonists and functional fragments of prolactin, for the therapeutic treatment, possibly also in combination with conventional therapies and drugs (eg testosterone/androgens and gonadotropins), of the disorders of Claim 13, and for treating or ameliorating dysfunctions associated with such disorders, when the prolactin level in blood is low or otherwise inadequate.

15. Kits containing a composition comprising **prolactin**, or a variant, analog, agonist or functional fragment of prolactin, in the appropriate doses and combinations, in one or more containers. This composition is for administering to the subjet, also, possibly, in combination with conventional therapies and drugs (eg testosterone/androgens and gonadotropins), for the therapeutic treatment of the disorders of claim 13, and for the treating or ameliorating dysfunctions associated with such desease/disorders, when the prolactin level in blood is low or otherwise inadequate. Any kit can include instructions for administering the composition to the subject and/or include a device for the same administration.
